# EUROPÄISCHE PATENTSCHRIFT

(11) **EP 4 353 205 B1**
(45) Veröffentlichungstag und Bekanntmachung des Hinweises auf die Patenterteilung: **22.07.2026**
(21) Anmeldenummer: 22200655.3
(22) Anmeldetag: 10.10.2022
(51) Int. Cl.: A61F 9/00, B05B 11/10

(54) **AUGENTROPFENSPENDER**
EYE DROP DISPENSER
DISTRIBUTEUR DE GOUTTES OCULAIRES

(43) Veröffentlichungstag der Anmeldung: 17.04.2024
(73) Patentinhaber: Aptar Radolfzell GmbH, 78315 Radolfzell (DE)
(72) Erfinder: Greiner-Perth, Jürgen, 78244 Gottmadingen (DE)
(74) Vertreter: Witte, Weller und Partner Patentanwälte mbB Stuttgart

(56) Entgegenhaltungen:
- US-A- 5 152 435
- US-A1- 2006 027 607
- US-A1- 2010 108 720

## Beschreibung

### ANWENDUNGSGEBIET UND STAND DER TECHNIK

Die Erfindung betrifft den Bereich der Augentropfenspender.

Augentropfenspender finden Verwendung zur Applikation von Flüssigkeit in das Auge eines Benutzers. Insbesondere kann es sich um eine Flüssigkeit zur Behandlung von trockenen oder geröteten Augen handeln. Aber auch andere Medikamente wie beispielsweise Antibiotika können mittels gattungsgemäßer und erfindungsgemäßer Augentropfenspender abgegeben werden.

Der üblichste Weg zur Verabreichung von Augentropfen liegt darin, die Augentropfen mittels eines Augentropfenspenders von oben in das Auge fallen zu lassen. Aus dem Augentropfenspender, beispielsweise eines Quetschflaschenspenders, wird vorsichtig eine Flüssigkeitsmenge abgegeben, die sich zunächst an einer Tropfenbildungsfläche des Spenders sammelt und sich bei ausreichender Größe hiervon löst und bestimmungsgemäß in das Auge des Benutzers fällt.

Die Verwendung eines solchen klassischen Augentropfenspenders birgt verschiedene Probleme. Zum einen fällt es manchem Benutzer schwer, das Auge zu treffen. Beim erforderlichen Blick nach oben ist es nicht gut abschätzbar, wann die Austragöffnung des Spenders sich direkt über dem Auge befindet. Dies kann auch dazu führen, dass der Augentropfenspender vom Benutzer sehr nah an das Auge herangeführt wird, was Kontakt und Schmerzen zur Folge haben kann. Auch das Auftreffen des üblicherweise recht großen Tropfens auf dem Auge wird von manchen Nutzern als unangenehm empfunden. Das Dokument US 2006/027607 offenbart eine Vorrichtung gemäß dem Oberbegriff des Anspruchs 1.

### AUFGABE UND LÖSUNG

Aufgabe der Erfindung ist es, Augentropfenspender und ein Betriebsverfahren hierfür zur Verfügung zu stellen, die in Hinblick auf Nutzungskomfort und/oder Applikationsgenauigkeiten bekannten Augentropfenspendern überlegen sind.

Zur Lösung dieser Aufgabe wird ein Augentropfenspender zur Applikation von pharmazeutischen Flüssigkeiten in ein Auge eines Nutzers vorgeschlagen, gemäß Anspruch 1.

Der Augentropfenspender weist einen Flüssigkeitsspeicher auf, der im verkaufsfertigen Zustand mit einer pharmazeutischen Flüssigkeit zur Applikation in ein Auge des Nutzers befüllt ist. Es kann sich insbesondere um eine pharmazeutische Flüssigkeiten zur Behandlung des erhöhten Augeninnendrucks (Glaukombehandlung) oder zur Behandlung des trockenen Auges und zur Behandlung von Allergien und Entzündungen handeln. Hierbei spielen insbesondere die Molekülgruppen Alpha-2 Agonisten, z. B. Brimonidin, Prostaglandinanaloga (Tafluprost, Latanoprost, Bimatoprost, Travoprost), Beta-Blocker, z. B. Timolol, und Carboanhydrasehemmer, z. B. Dorzolamid beziehungsweise Hyaluronsäureverbindungen, Filmbildner, z. B. Methylcelluloseverbindungen und Cyclosporin beziehungsweise Antihistaminika, z. B. Olopatadin und Levocabastin, Steroide, z. B. Loteprdnol und Dexamethason, sowie NSAID, z. B. Keterolac, eine Rolle. Wie eingangs bereits erläutert, kann es sich auch um ein anderweitiges Medikament wie beispielsweise ein Antibiotikum handeln.

Der erfindungsgemäße Augentropfenspender weist weiterhin eine Austragöffnung auf, durch die hindurch die Flüssigkeit ausgetragen werden kann, wobei der Austrag durch diese Austragöffnung vorzugsweise etwa in horizontaler Richtung erfolgt. Ein durch die Austragöffnung ausgetragener Tropfen wird gleichsam in das Auge geschossen.

Zur Förderung der Flüssigkeit aus dem Flüssigkeitsspeicher zur Austragöffnung ist eine Pumpeinrichtung vorgesehen, die insbesondere zur manuellen Betätigung vorgesehen ist. Es kann sich insbesondere um eine Kolbenpumpe handeln, deren Betätigung eine Pumpkammer verkleinert, so dass die in der Pumpkammer enthaltene und zuvor aus dem Flüssigkeitsspeicher angesogene Flüssigkeit unter Druck gesetzt wird und in Richtung der Austragöffnung und durch diese hinaus abgegeben wird.

Der Augentropfenspender weist zur Betätigung der Pumpeinrichtung zwei gegeneinander translativ bewegliche Teileinheiten auf, die zum Zwecke der Betätigung der Pumpeinrichtung und damit zur Bewirkung eines Austrags eines Flüssigkeitstropfens in einer Betätigungsrichtung aufeinander zu gedrückt werden können. Diese Betätigung erfolgt vorzugsweise gegen die Kraft einer Federeinrichtung, insbesondere einer zwischen den Teileinheiten wirkenden Schraubenfeder. Vorzugsweise handelt es sich um eine Haupteinheit, die bei Verwendung von einer Hand des Benutzers umgriffen wird, und um eine Betätigungseinheit, die mittels eines Fingers der Hand betätigt, insbesondere niedergedrückt, wird.

Vorzugsweise beinhaltet eine der beiden Teileinheiten den Flüssigkeitsspeicher und die andere der beiden Teileinheiten weist die Austragöffnung auf. Es sind aber auch Gestaltungen möglich, bei denen die Austragöffnung und der Flüssigkeitsspeicher an der gleichen Teileinheit vorgesehen sind.

Die andere Teileinheit ist dann im Wesentlichen nur ein Betätigungsdrücker zur Betätigung der Pumpeinrichtung.

Wie oben schon erwähnt, ist bei einem erfindungsgemäßen Augentropfenspender vorgesehen, dass die Flüssigkeit in Form einer zusammenhängenden definierten Flüssigkeitsmenge, im Kontext der Erfindung als Tropfen bezeichnet, gleichsam in das Auge geschossen wird, üblicherweise in etwa horizontaler Ausrichtung. Die Betätigung des Augentropfenspenders erfolgt jedoch in hiervon abweichender Richtung. Dies wird erzielt, indem die Austragöffnung derart ausgerichtet ist, dass ein Flüssigkeitsaustrag in einer Austragrichtung erfolgt, die von der Betätigungsrichtung der Teileinheiten abweicht. Vorzugsweise schließen die Betätigungsrichtung und die Austragrichtung einen Winkel zwischen 45° und 135° ein, insbesondere in etwa oder genau einen Winkel von 90°. Die Betätigungsrichtung, die zur Austragrichtung in genannter Weise angewinkelt ist, entspricht vorzugsweise auch der Haupterstreckungsrichtung des Augentropfenspender, die durch eine Mittelachse des Flüssigkeitsspeichers definiert ist.

Ein erfindungsgemäßer Augentropfenspender ist in der Handhabung besonders angenehm. Die Betätigung erfolgt, indem die Pumpeinrichtung durch in der Praxis in etwa vertikales Zusammendrücken der Teileinheiten betätigt wird, wobei der Flüssigkeitstropfen quer dazu abgegeben wird und in das Auge geschossen wird.

Damit das Auftreffen der Flüssigkeit im Auge für den Benutzer nicht unangenehm ist bzw. im Idealfalle nicht zu merken ist, wird vorgeschlagen, die Pumpeinrichtung derart auszulegen, dass mit einer vollständigen Betätigung, also mit Verlagerung der Teileinheiten aus einer beabstandeten Endlage in einer angenäherten Endlage, ein Flüssigkeitsvolumen von weniger als 25 µl in Richtung der Austragöffnung gefördert wird. Vorzugsweise ist das Flüssigkeitsvolumen noch geringer und liegt insbesondere vorzugsweise unter 20 µl. Diese Flüssigkeitsmenge bildet dann beim Austrag einen Flüssigkeitstropfen. Das Volumen von weniger als 25 µl, insbesondere von weniger als 20 µl, ist geringer als das üblicherweise über einen Spender zur Abgabe fallender Tropfen erzielte Tropfenvolumen. Es hat sich jedoch gezeigt, dass bei vielen Anwendungsfällen, insbesondere wenn eine vollständige Benetzung des Auges gewünscht ist, ein Volumen von 25 µl ausreichend ist, zumal die Flüssigkeitsmenge aufgrund der mit dem erfindungsgemäßen Augentropfenspender erzielbaren Applikationsgenauigkeit vollständig und nicht nur teilweise das Auge erreicht.

Das geringe Volumen des Flüssigkeitstropfens ist im Kontext des vorliegend vorgesehenen geschossenen Tropfens hilfreich, um das Auftreffen der Flüssigkeit im Auge für den Benutzer nicht unangenehm sein zu lassen.

Das genannte geringe Pumpvolumen der Pumpeinrichtungje Betätigungshub kann in Hinblick auf die Entlüftung einer Pumpkammer der Pumpeinrichtung bei Erstinbetriebnahme des Spenders, dem so genannten "Priming", problematisch sein. Ein geringes Pumpvolumen führt dazu, dass der durch Betätigung erzeugte Überdruck der im Lieferzustand in der Pumpkammer befindlichen Luft nicht ausreicht, um ein druckabhängig öffnendes Auslassventil zu öffnen. Um dieses Problem zu überwinden, ist es von Vorteil, wenn eine Pumpeinrichtung mit einer Entlüftungsfunktion vorgesehen ist. Bei einer solchen Pumpeinrichtung mit Entlüftungsfunktion wird gegen Ende des Betätigungshubes, also wenn die beiden Teileinheiten maximal einander angenähert sind, eine kommunizierende Verbindung zwischen dem Flüssigkeitsspeicher und der Pumpkammer geöffnet. Über diese Verbindung kann die komprimierte Luft zumindest teilweise aus der Pumpkammer entweichen, so dass im Zuge des darauffolgenden Rückhubs inkompressible Flüssigkeit in die Pumpkammer eingesogen wird. Wiederholte Betätigungen führen dazu, dass die Luft aus der Pumpkammer sukzessive vollständig verdrängt wird.

Neben dem Volumen ist auch die Geschwindigkeit, mit der der Tropfen abgegeben wird und mit der er auf dem Auge auftrifft, für die Frage relevant, ob das Auftreffen des Tropfens im Auge als unangenehm empfunden wird. Der Spender ist vorzugsweise dafür ausgelegt, die Flüssigkeit bei einer Betätigungsgeschwindigkeit von 35 mm/sec mit einer Geschwindigkeit von mindestens 2 m/sec auszutragen, insbesondere mit einer Geschwindigkeit von mindestens 3 m/sec, damit die Flugbahn des Tropfens weitgehend geradlinig erfolgt. Gleichzeitig liegt die Geschwindigkeit vorzugsweise nicht über 8 m/sec und vorzugsweise unter 6 m/sec, um das Auftreffen auf dem Auge als nicht unangenehm zu empfinden bzw. im Idealfalle nicht oder nur schwach merken zu können.

Die genannten Geschwindigkeiten liegen deutlich unter üblichen Austraggeschwindigkeiten von Flüssigkeitsspendern. Beispielsweise liegt die Austrittsgeschwindigkeit von Nasalspendern üblicherweise im Bereich zwischen 10 m/sec und 20 m/sec.

Neben dem Volumen des Tropfens und dem Zeitraum, über den dieses Volumen ausgetragen wird, ist auch die Gestaltung der Austragöffnung für die Austrittsgeschwindigkeit relevant. Für einen Augentropfenspender der erfindungsgemäßen Art wird es bevorzugt, wenn die Austrittsöffnung eine vergleichsweise große Querschnittsfläche an ihrer engsten Stelle aufweist. Der dortige lichte Querschnitt liegt vorzugsweise bei mindestens 0,2 mm², insbesondere vorzugsweise bei 0,5 mm² oder mehr. Vorzugsweise ist die Austrittsöffnung kreisrund und weist einen Durchmesser zwischen 0,5 mm und 0,8 mm auf.

Die Geometrie der Austragöffnung und des unmittelbaren Zulaufs zur Austragöffnung ist vorzugsweise derart gestaltet, dass die Flüssigkeit keinen Drall bezogen auf die Achse der Austragrichtung entwickelt, da ein solcher Drall die ungewünschte Zerstäubung der Flüssigkeit zur Folge haben könnte. Vorzugsweise erfolgt der Zulauf durch mindestens einen radialen Zulaufkanal, insbesondere vorzugsweise durch mindestens zwei über den Umfang gleichverteilte Zulaufkanäle, um einen drallfreien Zulauf zu ermöglichen.

Die Vermeidung eines Dralls und einer damit einhergehenden Neigung zur Zerstäubung der Flüssigkeit kann auch durch die Flüssigkeit selbst erreicht werden. Es hat sich gezeigt, dass Flüssigkeit mit einer gegenüber Wasser höheren Viskosität besonders geeignet ist, in Form eines Tropfens geschossen zu werden. Vorzugsweise ist der Flüssigkeitsspeicher daher mit einer Flüssigkeit mit einer Viskosität von mindestens 2 cP, vorzugsweise mit einer Viskosität zwischen 10 cP und 80 cP, insbesondere zwischen 30 cP und 50 cP, befüllt.

Um einen schussartig abgegebenen Tropfen des gewünschten Volumens zu erzeugen, wird es als vorteilhaft angesehen, dass der für den Austrag wirksame Teilhub der Pumpeinrichtung, also der Teilhub der Teileinheiten bei Betätigung, während dessen Durchlaufen der Austrag stattfindet, vorzugsweise höchstens 2 mm beträgt, insbesondere vorzugsweise 1,5 mm oder weniger. Im Falle einer Kolbenpumpe als Pumpeinrichtung ist das Volumen des Tropfens das Produkt aus diesem Teilhub und der wirksamen Kolbenfläche. Der vergleichsweise kleine wirksame Teilhub gewährleistet ein schnelles Durchlaufen und die dadurch erzielbare definierte Gestalt des geschossenen Tropfens.

Eine besonders bevorzugte Gestaltung der Pumpeinrichtung sieht vor, dass diese dafür ausgebildet ist, im Zuge der Verlagerung der beiden Teileinheiten ausgehend von einer unbetätigten Ruhestellung zunächst eine Leerhubstrecke und anschließend eine Wirkhubstrecke zu durchlaufen. Im Zuge der Verlagerung der Teileinheiten über die Leerhubstrecke findet keine Druckbeaufschlagung der Flüssigkeit in der Pumpkammer der Pumpeinrichtung statt. Vorzugsweise wird die Flüssigkeit aus der Pumpkammer während des Durchlaufens der Leerhubstrecke zurück in den Flüssigkeitsspeicher verdrängt. Erst wenn die Teileinheiten einander um die Leerhubstrecke angenähert sind, folgt die Wirkhubstrecke, über die hinweg die Verkleinerung der dann gegenüber dem Flüssigkeitsspeicher isolierten Pumpkammer und hieraus folgend der Austrag der Flüssigkeit in Form eines geschossenen Tropfens erfolgen.

Die genannte Leerhubstrecke ist für die Funktion des Augentropfenspenders nicht von zwingender Bedeutung. Der Austrag der Flüssigkeit kann auch direkt mit Beginn der Verlagerung der zwei Teileinheiten beginnen. Es hat sich allerdings gezeigt, dass die Bedienung von Benutzern als erheblich angenehmer empfunden wird, wenn über einen Teilhub, die genannte Leerhubstrecke, kein Austrag erfolgt, so dass während der Positionierung des Augentropfenspenders keine Gefahr eines versehentlichen Austrags gegeben ist. Zudem führt die Leerhubstrecke dazu, dass es erleichtert ist, die sich anschließende Wirkhubstrecke mit angemessen hoher Verlagerungsgeschwindigkeit der Teileinheiten zu durchfahren.

Die erforderliche manuelle Betätigungskraft während des Flüssigkeitsaustrags liegt bezogen auf eine Betätigungsgeschwindigkeit von 35 mm/sec vorzugsweise bei mindestens 15 Newton, insbesondere bei mindestens 18 Newton. Diese Kraft ist von der Rückstellfeder des Augentropfenspenders sowie vom Strömungswiderstand bestimmt, den die Flüssigkeit zwischen Pumpkammer und Austragöffnung erfährt und kann insbesondere auch bedingt sein durch ein im Strömungspfad befindliches Ventil und dessen erforderlichen Öffnungsdruck.

Sofern eine der Wirkhubstrecke vorgeschaltete Leerhubstrecke vorgesehen ist, ist dort die erforderliche Betätigungskraft geringer, bedingt primär dadurch, dass die Flüssigkeit in dieser Phase noch nicht strömt und somit der Strömungswiderstand die Betätigungskraft nicht beeinflusst. Die Betätigungskraft während des Durchfahrens der Leerhubstrecke beträgt bezogen auf eine Betätigungsgeschwindigkeit von 35 mm/sec vorzugsweise unter 15 Newton, insbesondere vorzugsweise unter 12 Newton.

Der Unterschied in der Betätigungskraft zwischen Leerhubstrecke und Wirkhubstrecke beträgt vorzugsweise bei üblicher Betätigungsgeschwindigkeit von 35 mm/sec mindestens 3 Newton, vorzugsweise mindestens 5 Newton. Dies bewirkt die haptische Wirkung eines Druckpunktes. Beim Verlagern der Teileinheiten kann der Beginn der Wirkhubstrecke einfach erspürt werden. Vorzugsweise ist der Übergang zwischen der Leerhubstrecke und der Wirkhubstrecke derart gestaltet, dass die erforderliche Betätigungskraft über eine Strecke von wenigstens 0,3 mm ansteigt, so dass die haptische Lokalisierung des Wechsels erleichtert ist.

Ein übliches Prozedere bei der Verwendung ist, dass der Benutzer den Spender vor dem Auge positioniert, manuell die Teileinheiten vorsichtig kraftbeaufschlagt, bis er den Übergang von Leerhubstrecke und Wirkhubstrecke lokalisiert hat, die Teileinheiten wieder etwas voneinander beabstandet und dann mit Teileinheiten mit definierter und ausreichend hoher Kraft zusammendrückt, so dass die Leerhubstrecke und die Wirkhubstrecke in schneller Folge durchfahren werden.

Die Länge der Leerhubstrecke beträgt vorzugsweise mehr als 1 mm, insbesondere mindestens 3 mm oder sogar mindestens 4 mm. Die Wirkhubstrecke ist vorzugsweise kürzer als die Leerhubstrecke. Vorzugsweise beträgt die Wirkhubstrecke höchstens 2 mm, insbesondere vorzugsweise höchstens 1,5 mm.

Die Wirkhubstrecke kann mit einer anschlagsartigen Begrenzung enden, über die hinaus die Teileinheiten nicht weiter einander angenähert werden können. Als vorteilhaft wird es allerdings angesehen, wenn sich an die Wirkhubstrecke eine weitere Resthubstrecke anschließt. Mit Erreichen der Resthubstrecke endet der Austrag, da der Überdruck in der Druckkammer entfällt, insbesondere vorzugsweise dadurch, dass eine kommunizierende Verbindung zwischen der Pumpkammer und dem Flüssigkeitsspeicher geschaffen wird, so dass Flüssigkeit bei über die Resthubstrecke fortgesetzt verkleinerter Pumpkammer in den Flüssigkeitsspeicher zurückfließen kann.

Insbesondere kann die genannte Resthubstrecke bei der Inbetriebnahme des Spenders und dem so genannten "Priming" hilfreich sein, also dem initialen Verdrängen von Luft aus der Pumpkammer, da bei nahezu vollständig komprimierter Pumpkammer die darin komprimierte Luft weitgehend vollständig aus der Pumpkammer in den Flüssigkeitsspeicher entweicht. Zudem verbessert die Resthubstrecke die Dosiergenauigkeit, da die Dosiergenauigkeit nicht durch einen ggf. nicht ausreichend definierten Anschlag am Ende der Wirkhubstrecke oder ein unwillkürliches Abbremsen der manuellen Betätigung durch den Benutzer aufgrund des nahenden Anschlages beeinträchtigt ist.

Zur technischen Realisierung einer Leerhubstrecke und einer Resthubstrecke kann beispielsweise vorgesehen sein, dass ein Kolben der Kolbenpumpe beweglich an der ihm zugeordneten Teileinheit vorgesehen ist, so dass über die Leerhubstrecke zunächst eine Verlagerung des Kolbens gegenüber dessen Teileinheit erfolgt, bevor anschließend nach Erreichen einer Endlage die genannte Teileinheit zusammen mit dem Kolben gegenüber der anderen Teileinheit und dem daran vorgesehenen Kolbenzylinder verlagert wird.

Vorteilhafter ist jedoch eine andere Gestaltung, bei der die Pumpeinrichtung ein Einlassventil aufweist, welches als Schieberventil ausgebildet ist. Ein solches Schieberventil zeichnet sich dadurch aus, dass ein Ventilschieber vorgesehen ist, der zu Beginn der Betätigung außerhalb eines Dosierkanals angeordnet ist und im Zuge der Betätigung in den Dosierkanal einfährt und innerhalb dessen verlagert wird. Das Schieberventil bildet vorzugsweise vorliegend ein Einlassventil der Pumpeinrichtung. Bis der Ventilschieber zusammen mit dem Dosierkanal den Einlass verschließt, ist die Pumpkammer mit dem Flüssigkeitsspeicher verbunden. Sobald der Ventilschieber in den Dosierkanal einfährt, wird der Pumpeinlass verschlossen und die fortgesetzte Betätigung führt zum Druckanstieg in der Flüssigkeit in der Pumpkammer und zum Flüssigkeitsaustrag. Der Eingang in den Dosierkanal ist vorzugsweise mit einer konischen Einführfläche versehen, die sich in Betätigungsrichtung wenigstens über 0,3 mm erstreckt. Diese führt zu einem sanften Einfangen des Ventilschiebers und zu einer guten haptischen Erfassbarkeit des Wechsels vom Leerhub in den Wirkhub.

Vorzugsweise sind die Komponenten derart dimensioniert, dass der Ventilschieber gegen Ende der Betätigung aus dem Dosierkanal wieder ausrückt, also wieder den Kontakt zur Wandung des Dosierkanals zumindest teilweise verliert, so dass die Pumpkammer und der Flüssigkeitsspeicher hierdurch verbunden werden. Die Teilstrecke der Verlagerung jenseits des Dosierkanals bildet die oben bezüglich ihrer Wirkung beschriebene Resthubstrecke und die eingangs genannte Entlüftungsfunktion.

Der Ventilschieber bildet eine Art Kolben im Dosierkanal. Zur Reduktion des Pumpkammervolumens ist jedoch vorzugsweise ein zusätzlicher, größerer Pumpenkolben vorgesehen, der zum Ventilschieber ortsfest ist und zumindest über die Wirkhubstrecke, vorzugsweise aber permanent, an einer zugehörigen Pumpenzylinderwandung anliegt, die wiederum vorzugsweise ortsfest zum Dosierkanal vorgesehen ist. Auch die umgekehrte Konfiguration ist möglich, also eine Ortsfestigkeit von Ventilschieber und Pumpenzylinderwandung zueinander sowie eine Ortfestigkeit von Dosierkanal und Pumpenkolben andererseits.

Vorzugsweise kann zwischen der Pumpkammer und der Austragöffnung ein Ventil vorgesehen sein. Insbesondere ist hier vorzugsweise ein Pumpenauslassventil vorgesehen, welches bei Unterdruck in der Pumpkammer schließt, so dass während des Rückhubs Flüssigkeit aus dem Flüssigkeitsspeicher in die Pumpkammer angesogen wird.

Weiterhin vorzugsweise weist der Augentropfenspender ein Austragventil auf, welches druckabhängig öffnet und welches der Austragöffnung unmittelbar vorgeschaltet ist. Dabei kann es sich um ein weiteres Ventil zusätzlich zu einem Pumpenauslassventil handeln. Das Austragventil kann jedoch auch das einzige Ventil zwischen der Pumpkammer und der Austragöffnung sein.

Das Austragventil öffnet bei einem definierten Mindestdruck und schließt, sobald der Flüssigkeitsdruck der zuströmenden Flüssigkeit unter diesen Mindestdruck fällt. Durch die Anordnung des Austragventils unmittelbar vor der Austragöffnung wird ein sehr definierter Beginn des Austrags und ein sehr definiertes Ende des Austrags bewirkt, wobei dies zur Bildung des geschossenen Tropfens von Vorteil ist. Vorzugsweise ist das Austragventil dafür ausgebildet, bei einem Überdruck in der Flüssigkeit von höchstens 6 bar zu öffnen, vorzugweise bei einem Überdruck in der Flüssigkeit von höchstens 4 bar. Diese vergleichsweise geringen Öffnungsdrücke sind für die Bildung eines geschossenen Tropfens ausreichend. Der erforderliche Druck, um das Austragventil vollständig zu öffnen, liegt vorzugsweise nur geringfügig über diesem minimalem Öffnungsdruck, insbesondere vorzugsweise nicht mehr als 1 bar über dem minimalen Öffnungsdruck.

Das Austragventil ist vorzugsweise mit einer Federeinrichtung ausgestattet, mittels derer ein Ventilkörper des Austragventils in eine Schließstellung gedrückt wird. Insbesondere die Auslegung dieser Feder bestimmt den genannten Öffnungsdruck.

Die Austragöffnung des erfindungsgemäßen Augentropfenspenders ist erfindungsgemäß zu einem Austrag in einer Austragrichtung ausgebildet, die gegenüber der Betätigungsrichtung angewinkelt ist. Vorzugsweise ist hier ein rechter Winkel vorgesehen. Der Augentropfenspender kann derart ausgestaltet sein, dass er bezogen auf die Achse der Betätigungsrichtung eine im wesentlichen rotationssymmetrische Formgebung aufweist. Die Austragöffnung ragt in einem solchen Falle nicht seitlich über die Mantelfläche der jeweiligen Teileinheit hinaus.

Denkbar ist jedoch auch die Anordnung der Austragöffnung am Ende eines sich radial nach außen erstreckenden Austragstutzens, vorzugsweise eines Austragsstutzens der einstückig mit einer Betätigungsfläche der Teileinheit ausgebildet ist.

Ein radial nach außen erstreckter Austragstutzen bildet eine gute Orientierungshilfe, um den Spender direkt in der richtigen Orientierung ergreifen zu können oder nach dem Ergreifen einfach ausrichten zu können. Insbesondere ist eine Außenfläche des Austragstutzens vorzugsweise kreisrund und konzentrisch mit der Austragöffnung.

Als weitere Maßnahme zur Erleichterung der Orientierung kann vorgesehen sein, dass die Austragöffnung optisch gut erfassbar ist, insbesondere durch einen Farbkontrast. Die Austragöffnung befindetsich bei bestimmungsgemäßer Nutzung des Augentropfenspenders vorzugsweise zwischen 2 cm und 6 cm vom Auge entfernt, so dass eine Fokussierung schwerfallen kann. Um die Ausrichtung zu erleichtern kann insbesondere vorgesehen sein, dass ein farblicher Kontrast zwischen dem die Austragöffnung bildenden Bauteil und einem Ventilkörper des Austragventils gegeben ist. Vorzugsweise ist der Ventilkörper gegenüber dem Austragsöffnungskörper dunkler. Insbesondere vorzugsweise weist der Ventilkörper eine von Weiß signifikant abweichende Farbe wie Blau, Rot, Grün oder Lila auf.

Eine bevorzugte Bauform des erfindungsgemäßen Augentropfenspenders sieht vor, dass bei Benutzung der Flüssigkeitsspeicher nach unten weist und die Pumpeinrichtung oberhalb des Flüssigkeitsspeichers angeordnet ist. Zum Ansaugen der Flüssigkeit aus dem Flüssigkeitsspeicher ist in diesem Falle vorzugsweise ein Steigrohr vorgesehen, welches von der Pumpeinrichtung in den Flüssigkeitsspeicher hineinragt. Bei dieser Bauform ist die zweite Teileinheit während der Nutzung vorzugsweise oberhalb der Pumpeinrichtung vorgesehen und bildet die Betätigungseinheit, die gegenüber der ersten Teileinheit, umfassend den Flüssigkeitsspeicher, niedergedrückt wird. Die Austragöffnung ist vorzugsweise an der zweiten Teileinheit vorgesehen. Zur Betätigung wird die erste Teileinheit, die gleichsam die Hauptteileinheit bildet, mit der Hand umgriffen und die zweite Teileinheit, die Betätigungseinheit, mittels eines Fingers dieser Hand niedergedrückt.

Ein andere Variante sieht vor, dass der Flüssigkeitsspeicher bei Nutzung oberhalb der Pumpeinrichtung vorgesehen ist und einen Teil der zweiten Teileinheit bildet, wobei hier diese zweite Teileinheit gegenüber der unter dieser befindlichen ersten Teileinheit niedergedrückt wird und die Flüssigkeit aus der Austragöffnung dieser ersten Teileinheit ausgetragen wird. Auch hierbei ist die erste Teileinheit vorzugweise die Hauptteileinheit, die zum Zwecke des Austrags mit einer Hand umgriffen wird, während die zweite Teileinheit die Betätigungseinheit bildet, die mittels eines Fingers niedergedrückt wird.

Neben dem Augentropfenspender selbst betrifft die Erfindung auch ein Betriebsverfahren hierfür. Gemäß diesem Verfahren werden zum Zwecke des Austrags die beiden Teileinheiten ausgehend von einer unbetätigten Ruhestellung in einer Betätigungsrichtung aufeinander zu bewegt, wodurch Flüssigkeit durch eine Pumpeinrichtung druckbeaufschlagt wird und hierdurch Flüssigkeit durch eine Austragöffnung in einer von der Betätigungsrichtung abweichenden Austragrichtung abgegeben wird. Dabei erfolgt die Flüssigkeitsabgabe in Form eines geschossenen Tropfens mit einem Tropfenvolumen zwischen 5 µl und 25 µl, der mit einer Geschwindigkeit zwischen 3 m/sec und 8 m/sec abgegeben wird.

Insbesondere kann vorgesehen sein, dass die beiden Teileinheiten ausgehend von einer unbetätigten Ruhestellung aufeinander zu bewegt werden, wobei zunächst über eine Leerhubstrecke von vorzugsweise mindestens 1 mm eine Pumpkammer des Augentropfenspenders kommunizierend mit dem Flüssigkeitsspeicher in Verbindung steht oder nicht verkleinert wird und wobei anschließend über eine sich an die Leerhubstrecke anschließende Wirkhubstrecke von vorzugsweise weniger als 2 mm die Pumpkammer verkleinert wird, während sie nicht mehr kommunizierend mit dem Flüssigkeitsspeicher in Verbindung steht, so dass der Flüssigkeitsdruck in der Pumpkammer erhöht wird und die Abgabe des Tropfens bewirkt wird.

### KURZBESCHREIBUNG DER ZEICHNUNGEN

Weitere Vorteile und Aspekte der Erfindung ergeben sich aus den Ansprüchen und aus der nachfolgenden Beschreibung eines bevorzugten Ausführungsbeispiels der Erfindung, das nachfolgend anhand der Figuren erläutert wird.
Fig. 1 zeigt einen erfindungsgemäßen Augentropfenspender in geschnittener Darstellung.
Fig. 2A bis 2D zeigen den Augentropfenspender in verschiedenen Phasen während der Betätigung.
Fig. 3 zeigt die Austragöffnung des Augentropfenspender und die Geometrie der Wandung innenseitig der Austragöffnung.

### DETAILLIERTE BESCHREIBUNG DER AUSFÜHRUNGSBEISPIELE

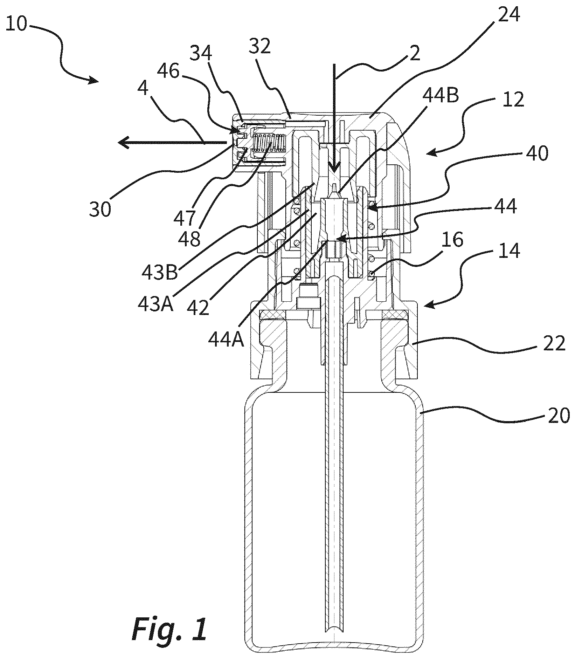

Fig. 1 zeigt einen erfindungsgemäßen Tropfenspender 10. Der Tropfenspender 10 verfügt über einen Flüssigkeitsspeicher 20 sowie einen am Flüssigkeitsspeicher 20 angebrachten Austragkopf mit einer Basis 22 sowie einem Betätigungsdrücker 24.

Die genannten Komponenten bilden zwei gegeneinander verlagerbare Teileinheiten 12, 14, wobei die Basis 22 des Austragkopfes und der Flüssigkeitsspeicher 20 eine Hauptteileinheit 14 bilden und wobei der Betätigungsdrücker 24 eine Betätigungsteileinheit 12 bildet.

Der Augentropfenspender 10 verfügt über eine Pumpeinrichtung 40, die als Kolbenpumpe ausgebildet ist und zwischen den Teileinheiten 12, 14 angeordnet ist, so dass durch Zusammendrücken der Teileinheiten 12, 14 Flüssigkeit in einer Pumpkammer 42 druckbeaufschlagt werden kann, um hierdurch Flüssigkeit durch die Austragöffnung 30 an der Teileinheit 12 austragen zu können.

Die Pumpeinrichtung 40 weist ein als Schiebeventil ausgebildetes Einlassventil 44 auf. Dieses verfügt über einen an der Basis 22 des Austragkopfes vorgesehenen Dosierkanal 44A sowie über einen am Betätigungsdrücker 24 vorgesehenen Ventilschieber 44B.

Die Pumpeinrichtung 40 verfügt weiterhin über ein Pumpenauslassventil, welches vorliegend durch ein der Austragöffnung 30 unmittelbar vorgeschaltetes Austragventil 46 gebildet ist. Dieses Austragventil 46 umfasst einen gegenüber einem Ventilsitz beweglichen Ventilkörper 47, der von einer Ventilfeder 48 in die geschlossene Stellung gedrückt wird.

Die Teileinheiten 12, 14 sind mittels einer Rückstellfeder 16 gegeneinander kraftbeaufschlagt. In der Ruhestellung der Fig. 1 nehmen sie ihren maximalen Abstand zueinander ein. In dieser Ruhestellung ist der Ventilschieber 44B des Einlassventils 44 noch nicht im Dosierkanal 44A angeordnet. Der Flüssigkeitsspeicher 20 und die Pumpkammer 42 sind dementsprechend in diesem Ausgangszustand noch miteinander kommunizierend verbunden.

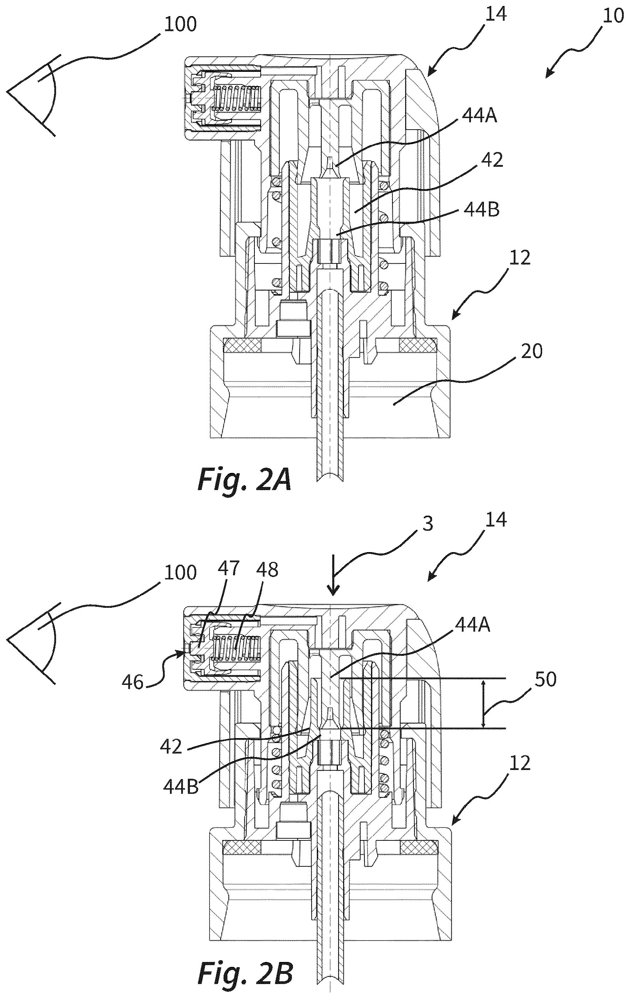

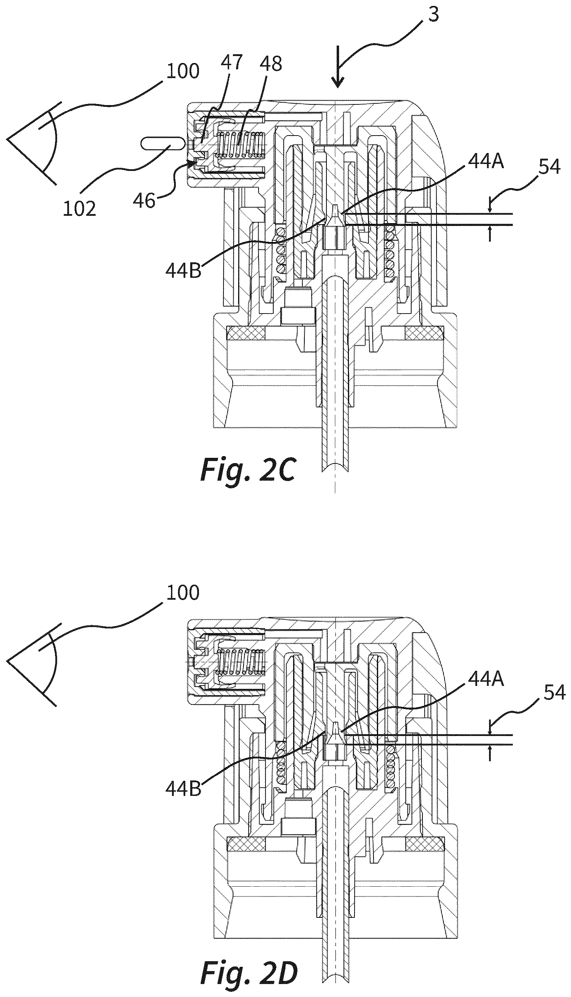

Anhand der Fig. 2A bis 2D wird die Funktionsweise des Augentropfenspenders 10 erläutert.

Fig. 2A zeigt nochmals den Ruhezustand der Fig. 1. Zusätzlich ist hieraus ersichtlich, dass der Augentropfenspender 10 zur bestimmungsgemäßen Abgabe von Augentropfen auf Höhe des Auges 100 und etwa 3 bis 5 cm von diesem beabstandet positioniert wird.

Ausgehend von dieser Stellung und unter der Annahme, dass die Pumpkammer 42 zu diesem Zeitpunkt bereits vollständig mit Flüssigkeit befüllt ist, führt eine Betätigung in Richtung des Pfeils 3, siehe Übergang von Fig. 2A zu Fig. 2B, zunächst nicht zu einem Austrag von Flüssigkeit. Die Teileinheit 14 wird zwar niedergedrückt beziehungsweise die Teileinheit 12 angehoben. Der Ventilschieber 44B gelangt jedoch in dieser Phase noch nicht in Kontakt mit der Wandung des Dosierkanals 44A. Daher kann in dieser Phase der Betätigung Flüssigkeit aus der Pumpkammer 42 während der Verkleinerung des Innenvolumens der Pumpkammer 42 noch zurück in den Flüssigkeitsspeicher 20 strömen. Zwischen der Stellung der Fig. 2A und der Stellung der Fig. 2B, in der die Teileinheit 12 etwa um 4 mm niedergedrückt ist, kann der Benutzer die Teileinheiten frei gegeneinander bewegen, ohne dass dies Auswirkungen auf einen Flüssigkeitsaustrag hat. Dies ermöglicht ihm auch, die Zwischenstellung der Fig. 2B, in der der Ventilschieber 44B in Kontakt mit dem Dosierkanal 44A gelangt, genau zu lokalisieren. Die Teilstrecke, um die die Teileinheiten 12, 14 ohne Austragswirkung gegeneinander beweglich sind, stellt die sogenannte Leerhubstrecke 50 dar.

Sobald der Augentropfenspender 10 richtig gegenüber dem Auge 100 positioniert ist und der Benutzer sich über die Lage der Teileinheiten 12, 14 im Stadium der Fig. 2B einen Eindruck verschafft hat, drückt der Benutzer die Teileinheit 14 nieder bis in die Position der Fig. 2C. Hierbei dichtet zunächst in der Position der Fig. 2B der Ventilschieber 44B mit dem Dosierkanal 44A ab, wodurch die Pumpkammer 42 gegenüber dem Flüssigkeitsspeicher isoliert ist. Die fortgesetzte Bewegung entlang des in Fig. 2C dargestellten Wirkhubs führt nun dazu, dass sich der Druck in der Pumpkammer 42 aufgrund der Inkompressibilität der Flüssigkeit erhöht und somit das Austragventil 46 öffnet, indem der Ventilkörper 47 gegen die Kraft der Ventilfeder 48 bezogen auf Fig. 2C nach rechts gedrückt wird. Wie anhand der Fig. 2C ersichtlich, führt dies zur schussartigen Abgabe eines Tropfens 102. Der Tropfen 102 wird ohne Zerstäubung etwa mit einer Geschwindigkeit von 1,5 m/s horizontal ausgetragen und trifft unmittelbar danach auf das Auge 100. Aufgrund der horizontalen und geradlinigen Bewegungsrichtung des Tropfens 102 ist dieser sehr präzise.

Sobald der Ventilschieber 44B am unteren Ende den Dosierkanal 44A verlässt, dargestellt durch den Zustand der Fig. 2C, ist eine Kommunikation zwischen der Pumpkammer 42 und dem Flüssigkeitsspeicher 20 am Ventilschieber 44B vorbei wieder möglich. Schlagartig bricht der Überdruck in der Pumpkammer 42 zusammen und ebenso schlagartig schließt das Austragventil 46. Dies führt zu einem präzisen Abriss des Flüssigkeitstropfens 102 von der Austragöffnung 30. Durch den schlagartigen Abriss des Flüssigkeitstropfens 102 wird auch wirksam der Verbleib von Restflüssigkeit außenseitig der Austragöffnung 30 verhindert.

Entfällt nach Betätigung abschließend die auf die Teileinheiten 12, 14 ausgeübte Kraft, so kehren die Teileinheiten 12, 14 unter Wirkung der Rückstellfeder 16 in ihre Ausgangslage zurück. Während der Ventilschieber 44B hierbei den Dosierkanal 44A erneut durchquert, ist der Zufluss in die Pumpkammer 42 kurz unterbrochen. Es baut sich hier kurzzeitig ein Unterdruck in der Pumpkammer 42 auf. Sobald der Ventilschieber 44B jedoch den Dosierkanal 44A am oberen Ende wieder verlässt und die kommunizierende Verbindung zum Flüssigkeitsspeicher 20 wieder geschaffen ist, wird Flüssigkeit eingesogen und füllt die Pumpkammer 42.

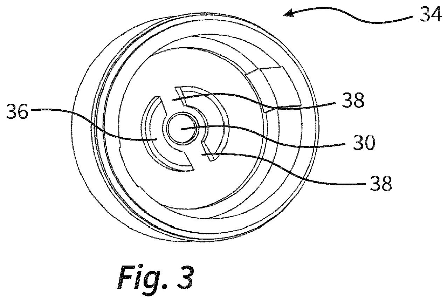

Fig. 3 zeigt ein Gehäusebauteil 34, in dem die Austragöffnung 30 vorgesehen ist. Zu erkennen ist, dass die Austragöffnung vergleichsweise groß ist, vorliegend vorzugsweise einen Durchmesser zwischen 0,5 und 0,8 mm aufweist. Dieser relativ große Durchmesser dient dem Zweck, die auszutragende Flüssigkeit nicht zu stark zu beschleunigen und zudem eine Zerstäubung zu vermeiden.

Ebenfalls zum Zwecke der Vermeidung einer Zerstäubung ist vorgesehen, dass die Flüssigkeit durch zwei Zulauföffnungen 38 in einem innenseitigen die Austragöffnung30 umgebenden Steg 36 zur Austragöffnung 30 gelangt, wobei die Zulauföffnungen 38 jeweils radial ausgerichtet und einander gegenüberliegend angeordnet sind. Auf diese Weise wird ebenfalls begünstigt, dass die Flüssigkeit beim Austritt nicht zerstäubt wird.

## Patentansprüche

1. Augentropfenspender (10) zur Applikation von pharmazeutischen Flüssigkeiten in ein Auge eines Nutzers mit den folgenden Merkmalen:
a. der Augentropfenspender (10) weist einen Flüssigkeitsspeicher (20) und eine Austragöffnung (30) auf, und
b. der Augentropfenspender (10) weist eine Pumpeinrichtung (40) auf, mittels derer Flüssigkeit aus dem Flüssigkeitsspeicher (20) zur Austragöffnung (30) gefördert werden kann, und
c. der Augentropfenspender (10) weist zwei gegeneinander translativ bewegliche Teileinheiten (12, 14) auf, die zum Zwecke der Betätigung der Pumpeinrichtung (40) und zur Bewirkung eines Austrags eines Flüssigkeitstropfens in einer Betätigungsrichtung (2) aufeinander zu gedrückt werden können, und
d. die Austragöffnung (30) ist derart ausgerichtet, dass ein Flüssigkeitsaustrag in einer Austragrichtung (4) erfolgt, die von der Betätigungsrichtung (2) abweicht und vorzugsweise mit der Betätigungsrichtung einen Winkel zwischen 45° und 135° einschließt, vorzugsweise zwischen 80° und 100°, und
e. die Pumpeinrichtung (40) ist als Kolbenpumpe ausgebildet und weist eine Pumpkammer (42) auf, die durch Verlagerung der beiden Teileinheiten (12, 14) im Zuge eines Betätigungshubs verkleinerbar ist, um in der Pumpkammer (42) befindliche Flüssigkeit mit Druck zu beaufschlagen,
**gekennzeichnet durch** das folgende Merkmal:
f. der Augentropfenspender ist zur Flüssigkeitsabgabe in Form eines geschossenen Tropfens mit einem Tropfenvolumen zwischen 5 µl und 25 µl ausgebildet, der mit einer Geschwindigkeit zwischen 3 m/sec und 8 m/sec abgegeben wird, und
g. zur Erzielen eines präzisen Abrisses des Tropfens von der Austragöffnung weist die Pumpeinrichtung (40) eine Entlüftungsfunktion auf, mittels derer gegen Ende des Betätigungshubs eine kommunizierende Verbindung zwischen dem Flüssigkeitsspeicher (20) und der Pumpkammer (42) geschaffen wird.

2. Augentropfenspender (10) nach Anspruch 1 mit dem folgenden weiteren Merkmal:
a. die Pumpeinrichtung (40) weist ein Pumpvolumenje Betätigungshub von weniger als 25 µl auf, vorzugsweise von weniger als 20 µl.

3. Augentropfenspender (10) nach einem der vorstehenden Ansprüche mit dem folgenden weiteren Merkmal:
a. die Austragöffnung (30) weist einen lichten Querschnitt von mindestens 0,2 mm² auf, vorzugsweise von mindestens 0,5 mm².

4. Augentropfenspender (10) nach einem der vorstehenden Ansprüche mit dem folgenden weiteren Merkmal:
a. der Austragöffnung (30) ist mindestens ein radial zur Austragrichtung erstreckter Zulaufkanal (38) zugeordnet, mittels dessen Flüssigkeit drallfrei der Austragöffnung (30) zugeführt werden kann.

5. Augentropfenspender (10) nach einem der vorstehenden Ansprüche mit den folgenden weiteren Merkmalen:
a. die Pumpeinrichtung (40) ist derart ausgebildet, dass im Zuge der Verlagerung der beiden Teileinheiten (12, 14) ausgehend von einer unbetätigten Ruhestellung zunächst eine Leerhubstrecke (50) und anschließend eine Wirkhubstrecke (52) durchlaufen werden,
b. im Zuge der Verlagerung der Teileinheiten (12, 14) über die Leerhubstrecke (50) erfolgt keine Druckbeaufschlagung der Flüssigkeit in einer Pumpkammer (42) der Pumpeinrichtung (40), und
c. im Zuge der anschließenden Verlagerung der Teileinheiten (12, 14) über die Wirkhubstrecke (52) steigt der Flüssigkeitsdruck in der Pumpkammer (42) und die Flüssigkeit wird aus der Pumpkammer (42) in Richtung der Austragöffnung (30) abgegeben.
vorzugsweise mit mindestens einem der folgenden zusätzlichen Merkmale:
d. die Leerhubstrecke (50) weist eine Länge von mindestens 3 mm auf, vorzugsweise von mindestens 4 mm, und/oder
e. die Wirkhubstrecke (52) weist eine Länge von höchstens 2 mm auf, vorzugsweise von höchstens 1,5 mm.

6. Augentropfenspender (10) nach Anspruch 5 mit dem folgenden weiteren Merkmal:
a. eine Betätigungskraft, die zum Zusammendrücken der Teileinheiten über die Leerhubstrecke aufzuwenden ist, liegt unterhalb von 15 Newton und eine Betätigungskraft am Übergang zwischen Leerhubstrecke und Wirkhubstrecke liegt oberhalb von 15 Newton, wobei vorzugsweise die Betätigungskraft für die Leerhubstrecke unterhalb von 12 Newton liegt und/oder die Betätigungskraft am Übergang zwischen Leerhubstrecke und Wirkhubstrecke oberhalb von 18 Newton liegt.

7. Augentropfenspender (10) nach einem der vorstehenden Ansprüche mit den folgenden weiteren Merkmalen:
a. die Pumpeinrichtung (40) weist ein Einlassventil (44) auf, welches als Schieberventil ausgebildet ist, wobei das Schieberventil eine Dosierstrecke in Form eines zylindrischen Dosierkanals (44A) sowie einen Ventilschieber (44B) aufweist, mittels dessen die Pumpkammer (42) eingangsseitig verschließbar ist, indem der Ventilschieber (44B) in den Dosierkanal (44A) einrückt, und
b. in einer unbetätigten Ruhstellung der beiden Teileinheiten (12, 14) befindet sich der Ventilschieber (44B) außerhalb des Dosierkanals (44A) und fährt im Zuge der Betätigung an einer Einfahrseite in den Dosierkanal (44A) ein,
vorzugsweise mit dem folgenden zusätzlichen Merkmal:
c. vor Erreichen einer betätigten Endstellung fährt der Ventilschieber (44B) an einer Ausfahrseite aus dem Dosierkanal (44A) heraus.

8. Augentropfenspender (10) nach Anspruch 7 mit den folgenden weiteren Merkmalen:
a. die Pumpeinrichtung (40) weist eine Pumpkammer (42) auf, die durch einen Pumpenzylinder (43A) und einen im Pumpenzylinder (43A) beweglichen Pumpenkolben (43B) begrenzt wird, und
b. der Ventilschieber (44B) des Einlassventils (44) ist ortsfest zum Pumpenkolben (43B) vorgesehen, und
c. der zylindrische Dosierkanal (44A) des Einlassventils (44) ist ortsfest zum Pumpenzylinder (43A) vorgesehen.

9. Augentropfenspender (10) nach einem der vorstehenden Ansprüche mit dem folgenden weiteren Merkmal:
a. der Augentropfenspender (10) weist ein Austragventil (46) auf, welches druckabhängig öffnet und welches der Austragöffnung (30) unmittelbar vorgeschaltet ist,
vorzugsweise mit den folgenden zusätzlichen Merkmalen:
b. das Austragventil (46) des Augentropfenspender (10) wird durch ein Pumpen-Auslassventil gebildet, welches die Pumpkammer (42) ausgangsseitig begrenzt, und/oder
c. das Austragventil (46) weist eine Federeinrichtung auf, mittels derer ein Ventilkörper (47) in eine Schließstellung gedrückt wird, wobei das Auslassventil derart ausgelegt ist, dass ein Flüssigkeitsdruck von weniger als 6 bar, vorzugsweise von weniger als 4 bar, das Austragventil (46) öffnet.

10. Augentropfenspender (10) nach einem der vorstehenden Ansprüche mit den folgenden weiteren Merkmalen:
a. eine erste der beiden Teileinheiten (14) bildet eine Hauptteileinheit, die zum Zwecke des Austrags mit einer Hand umgriffen wird, und
b. eine zweite der beiden Teileinheiten (12) bildet eine Betätigungsteileinheit, die an einer endseitigen Stirnfläche über eine mittels des Fingers der Hand niederdrückbare Betätigungsfläche (12A) verfügt,
vorzugsweise mit dem folgenden zusätzlichen Merkmal:
c. die Pumpeinrichtung (40) ist mit einem in den Flüssigkeitsspeicher (20) hineinragenden Steigrohr versehen.

11. Augentropfenspender (10) nach einem der vorstehenden Ansprüche mit einem der folgenden weiteren Merkmale:
a. die Austragöffnung (30) ist an der zweiten der beiden Teileinheiten (12) vorgesehen, oder
b. die Austragöffnung (30) ist an der ersten der beiden Teileinheiten (14) vorgesehen.

12. Augentropfenspender (10) nach einem der vorstehenden Ansprüche mit den folgenden weiteren Merkmalen:
a. der Augentropfenspender weist eine im Wesentlichen rotationssymmetrische Außenkontur auf, wobei im Bereich der Austragöffnung ein sich radial nach außen erstreckender Austragstutzen vorgesehen ist, an dessen distalem Ende die Austragöffnung vorgesehen ist, und/oder
b. die Austragöffnung ist durch einen Farbkontrast hervorgehoben, wobei insbesondere ein von der Austragöffnung durchdrungenes Bauteil eine von einem durch die Austragöffnung erkennbaren Ventilkörper abweichende Farbgebung aufweist.

## Claims

1. Eye drop dispenser (10) for administration of pharmaceutical fluids to an eye of a user having the following features:
a. the eye drop dispenser (10) has a fluid reservoir (20) and a discharge opening (30) and
b. the eye drop dispenser (10) has a pump device (40) by means of which fluid can be conveyed from the fluid reservoir (20) to the discharge opening (30) and
c. the eye drop dispenser (10) has two subunits (12, 14) which can be moved in translation with respect to each other and which in order to activate the pump device (40) and in order to bring about a discharge of a fluid drop can be pressed in an activation direction (2) towards each other, and
d. the discharge opening (30) is oriented in such a manner that a fluid discharge is carried out in a discharge direction (4) which differs from the activation direction (2) and preferably encloses with the activation direction an angle between 45° and 135°, preferably between 80° and 100°, and
e. the pump device (40) is in the form of a piston pump and has a pump chamber (42) which by displacing the two subunits (12, 14) can be reduced during an actuation travel in order to apply pressure to fluid located in the pump chamber (42),
**characterized by** the following feature:
f. the eye drop dispenser is configured for fluid discharge in the form of a jetted drop with a drop volume between 5 µl and 25 µl which is discharged at a speed between 3 m/sec and 8 m/sec, and
g. for achieving a precise breakaway of the drop from the discharge opening, the pump device (40) has a venting function by means of which towards the end of the actuation travel a communicating connection is provided between the fluid reservoir (20) and the pump chamber (42).

2. Eye drop dispenser (10) according to Claim 1, having the following additional feature:
a. the pump device (40) has a pump volume per actuation travel of less than 25 µl, preferably less than 20 µl.

3. Eye drop dispenser (10) according to one of the preceding claims, having the following additional feature:
a. the discharge opening (30) has a clear cross section of at least 0.2 mm², preferably at least 0.5 mm².

4. Eye drop dispenser (10) according to one of the preceding claims, having the following additional feature:
a. the discharge opening (30) is associated with at least one supply channel (38) which extends radially with respect to the discharge direction and by means of which fluid can be supplied in a swirl-free manner to the discharge opening (30).

5. Eye drop dispenser (10) according to one of the preceding claims, having the following additional features:
a. the pump device (40) is configured in such a manner that, during the displacement of the two subunits (12, 14) from a non-activated idle position, first an idle travel path (50) and subsequently an active travel path (52) are passed through,
b. during the displacement of the subunits (12, 14) over the idle travel path (50) no pressure is applied to the fluid in a pump chamber (42) of the pump device (40), and
c. during the subsequent displacement of the subunits (12, 14) over the active travel path (52) the fluid pressure in the pump chamber (42) rises and the fluid is discharged from the pump chamber (42) in the direction of the discharge opening (30),
preferably having at least one of the following additional features:
d. the idle travel path (50) has a length of at least 3 mm, preferably at least 4 mm, and/or
e. the active travel path (52) has a length of a maximum of 2 mm, preferably a maximum of 1.5 mm.

6. Eye drop dispenser (10) according to Claim 5, having the following additional feature:
a. an activation force which is intended to be applied in order to press together the subunits over the idle travel path is below 15 newtons and an activation force at the transition between the idle travel path and the active travel path is above 15 newtons, wherein the activation force for the idle travel path is preferably below 12 newtons and/or the activation force at the transition between the idle travel path and the active travel path is preferably above 18 newtons.

7. Eye drop dispenser (10) according to one of the preceding claims, having the following additional features:
a. the pump device (40) has an inlet valve (44) which is in the form of a sliding valve, wherein the sliding valve has a metering path in the form of a cylindrical metering channel (44A) and a valve slide (44B) by means of which the pump chamber (42) can be closed at the inlet side by the valve slide (44B) being introduced into the metering channel (44A) and,
b. in a non-activated idle position of the two subunits (12, 14), the valve slide (44B) is located outside the metering channel (44A) and moves during the activation into the metering channel (44A) at an inlet side,
preferably having the following additional feature:
c. before reaching an activated end position, the valve slide (44B) moves at an outlet side out of the metering channel (44A).

8. Eye drop dispenser (10) according to Claim 7, having the following additional features:
a. the pump device (40) has a pump chamber (42) which is delimited by a pump cylinder (43A) and a pump piston (43B) which can be moved in the pump cylinder (43A) and
b. the valve slide (44B) of the inlet valve (44) is provided in a fixed manner with respect to the pump piston (43B) and
c. the cylindrical metering channel (44A) of the inlet valve (44) is provided in a fixed manner with respect to the pump cylinder (43A).

9. Eye drop dispenser (10) according to one of the preceding claims, having the following additional feature:
a. the eye drop dispenser (10) has a discharge valve (46) which opens in a manner dependent on pressure and which is arranged directly upstream of the discharge opening (30),
preferably having the following additional features:
b. the discharge valve (46) of the eye drop dispenser (10) is formed by a pump outlet valve which delimits the pump chamber (42) at the outlet side, and/or
c. the discharge valve (46) has a resilient device by means of which a valve member (47) is pressed into a closure position, wherein the outlet valve is configured in such a manner that a fluid pressure of less than 6 bar, preferably of less than 4 bar, opens the discharge valve (46).

10. Eye drop dispenser (10) according to one of the preceding claims, having the following additional features:
a. a first of the two subunits (14) forms a main subunit which for the purposes of discharge is gripped with a hand and
b. a second of the two subunits (12) forms an activation subunit which at an end front face has an actuation face (12A) which can be pressed down by means of a finger of one hand,
preferably having the following additional feature:
c. the pump device (40) is provided with a riser pipe which protrudes into the fluid reservoir (20).

11. Eye drop dispenser (10) according to one of the preceding claims, having one of the following additional features:
a. the discharge opening (30) is provided on the second of the two subunits (12), or
b. the discharge opening (30) is provided on the first of the two subunits (14).

12. Eye drop dispenser (10) according to one of the preceding claims, having the following additional features:
a. the eye drop dispenser has a substantially rotationally symmetrical outer contour, wherein in the region of the discharge opening a radially outwardly extending discharge nozzle is provided, at the distal end of which the discharge opening is provided, and/or
b. the discharge opening is highlighted by a colour contrast, wherein in particular a component through which the discharge opening extends has a colour which differs from a valve member which can be identified through the discharge opening.

## Revendications

1. Distributeur de gouttes oculaires (10) pour l'application de liquides pharmaceutiques dans un œil d'un utilisateur, présentant les caractéristiques suivantes :
a. le distributeur de gouttes oculaires (10) présente un réservoir de liquide (20) et une ouverture de sortie (30), et
b. le distributeur de gouttes oculaires (10) présente un dispositif de pompe (40), au moyen duquel du liquide peut être acheminé du réservoir de liquide (20) vers l'ouverture de sortie (30), et
c. le distributeur de gouttes oculaires (10) présente deux unités partielles (12, 14) mobiles en translation l'une par rapport à l'autre, qui peuvent être pressées l'une contre l'autre dans une direction d'actionnement (2) aux fins de l'actionnement du dispositif de pompe (40) et pour provoquer une émission d'une goutte de liquide, et
d. l'ouverture de sortie (30) est orientée de telle sorte qu'une émission de liquide s'effectue dans une direction d'émission (4) qui s'écarte de la direction d'actionnement (2) et qui forme de préférence avec la direction d'actionnement un angle compris entre 45° et 135°, de préférence entre 80° et 100°, et
e. le dispositif de pompe (40) est réalisé sous la forme d'une pompe à piston et présente une chambre de pompe (42), qui peut être réduite par déplacement des deux unités partielles (12, 14) au cours d'une course d'actionnement afin de soumettre à pression le liquide se trouvant dans la chambre de pompe (42),
**caractérisé par** la particularité suivante :
f. le distributeur de gouttes oculaires est réalisé pour la délivrance de liquide sous la forme d'une goutte projetée ayant un volume de goutte compris entre 5 µl et 25 µl, qui est délivrée avec une vitesse comprise entre 3 m/s et 8 m/s, et
g. pour obtenir une séparation précise de la goutte de l'ouverture de sortie, le dispositif de pompe (40) présente une fonction d'évent, au moyen de laquelle, vers la fin de la course d'actionnement, une liaison communicante entre le réservoir de liquide (20) et la chambre de pompe (42) est établie.

2. Distributeur de gouttes oculaires (10) selon la revendication 1 présentant la caractéristique supplémentaire suivante :
a. le dispositif de pompe (40) présente un volume de pompe par course d'actionnement inférieur à 25 µl, de préférence inférieur à 20 µl.

3. Distributeur de gouttes oculaires (10) selon l'une quelconque des revendications précédentes présentant la caractéristique supplémentaire suivante :
a. l'ouverture de sortie (30) présente une section libre d'au moins 0,2 mm², de préférence d'au moins 0,5 mm².

4. Distributeur de gouttes oculaires (10) selon l'une quelconque des revendications précédentes présentant la caractéristique supplémentaire suivante :
a. au moins un canal d'alimentation (38) s'étendant radialement par rapport à la direction d'émission est associé à l'ouverture de sortie (30), au moyen duquel du liquide peut être amené à l'ouverture de sortie (30) sans tourbillon.

5. Distributeur de gouttes oculaires (10) selon l'une quelconque des revendications précédentes présentant les caractéristiques supplémentaires suivantes :
a. le dispositif de pompe (40) est réalisé de telle sorte que, lors du déplacement des deux unités partielles (12, 14) à partir d'une position de repos non actionnée, une course à vide (50) est d'abord parcourue, puis une course active (52),
b. au cours du déplacement des unités partielles (12, 14) sur la course à vide (50), aucune mise sous pression du liquide dans une chambre de pompe (42) du dispositif de pompe (40) n'a lieu, et
c. au cours du déplacement subséquent des unités partielles (12, 14) sur la course active (52), la pression du liquide dans la chambre de pompe (42) augmente et le liquide est émis de la chambre de pompe (42) en direction de l'ouverture de sortie (30),
de préférence avec au moins une des caractéristiques supplémentaires suivantes :
d. la course à vide (50) présente une longueur d'au moins 3 mm, de préférence d'au moins 4 mm, et/ou
e. la course active (52) présente une longueur d'au plus 2 mm, de préférence d'au plus 1,5 mm.

6. Distributeur de gouttes oculaires (10) selon la revendication 5 présentant la caractéristique supplémentaire suivante :
a. une force d'actionnement qui doit être appliquée pour comprimer les unités partielles sur la course à vide est inférieure à 15 newtons et une force d'actionnement à la transition entre la course à vide et la course active est supérieure à 15 newtons, la force d'actionnement pour la course à vide étant de préférence inférieure à 12 newtons et/ou la force d'actionnement à la transition entre la course à vide et la course active étant supérieure à 18 newtons.

7. Distributeur de gouttes oculaires (10) selon l'une quelconque des revendications précédentes présentant les caractéristiques supplémentaires suivantes :
a. le dispositif de pompe (40) présente une soupape d'admission (44), qui est réalisée sous la forme d'une soupape à tiroir, la soupape à tiroir présentant une section de dosage sous la forme d'un canal de dosage cylindrique (44A) ainsi qu'un tiroir de soupape (44B), au moyen duquel la chambre de pompe (42) peut être fermée du côté de l'admission par le fait que le tiroir de soupape (44B) s'engage dans le canal de dosage (44A), et
b. dans une position de repos non actionnée des deux unités partielles (12, 14), le tiroir de soupape (44B) se trouve à l'extérieur du canal de dosage (44A) et entre, au cours de l'actionnement, dans le canal de dosage (44A) d'un côté d'entrée,
de préférence avec la caractéristique supplémentaire suivante :
c. avant d'atteindre une position finale actionnée, le tiroir de soupape (44B) sort du canal de dosage (44A) d'un côté de sortie.

8. Distributeur de gouttes oculaires (10) selon la revendication 7 présentant les caractéristiques supplémentaires suivantes :
a. le dispositif de pompe (40) présente une chambre de pompe (42), qui est délimitée par un cylindre de pompe (43A) et un piston de pompe (43B) mobile dans le cylindre de pompe (43A), et
b. le tiroir de soupape (44B) de la soupape d'admission (44) est prévu fixe par rapport au piston de pompe (43B), et
c. le canal de dosage cylindrique (44A) de la soupape d'admission (44) est prévu fixe par rapport au cylindre de pompe (43A).

9. Distributeur de gouttes oculaires (10) selon l'une quelconque des revendications précédentes présentant la caractéristique supplémentaire suivante :
a. le distributeur de gouttes oculaires (10) présente une soupape de sortie (46), qui s'ouvre en fonction de la pression et qui est disposée immédiatement en amont de l'ouverture de sortie (30),
de préférence avec les caractéristiques supplémentaires suivantes :
b. la soupape de sortie (46) du distributeur de gouttes oculaires (10) est formée par une soupape d'échappement de pompe, qui délimite la chambre de pompe (42) du côté de la sortie, et/ou
c. la soupape de sortie (46) présente un dispositif de ressort, au moyen duquel un corps de soupape (47) est poussé dans une position de fermeture, la soupape d'échappement étant conçue de telle sorte qu'une pression de liquide inférieure à 6 bar, de préférence inférieure à 4 bar, ouvre la soupape de sortie (46).

10. Distributeur de gouttes oculaires (10) selon l'une quelconque des revendications précédentes présentant les caractéristiques supplémentaires suivantes :
a. une première des deux unités partielles (14) forme une unité partielle principale, qui est saisie d'une main aux fins de l'émission, et
b. une deuxième des deux unités partielles (12) forme une unité partielle d'actionnement, qui présente sur une surface frontale d'extrémité une surface d'actionnement (12A) pouvant être enfoncée au moyen du doigt de la main,
de préférence avec la caractéristique supplémentaire suivante :
c. le dispositif de pompe (40) est muni d'un tube plongeur pénétrant dans le réservoir de liquide (20).

11. Distributeur de gouttes oculaires (10) selon l'une quelconque des revendications précédentes présentant l'une des caractéristiques supplémentaires suivantes :
a. l'ouverture de sortie (30) est prévue sur la deuxième des deux unités partielles (12), ou
b. l'ouverture de sortie (30) est prévue sur la première des deux unités partielles (14).

12. Distributeur de gouttes oculaires (10) selon l'une quelconque des revendications précédentes présentant les caractéristiques supplémentaires suivantes :
a. le distributeur de gouttes oculaires présente un contour extérieur essentiellement à symétrie de rotation, un embout de sortie s'étendant radialement vers l'extérieur étant prévu dans la zone de l'ouverture de sortie, l'ouverture de sortie étant prévue à l'extrémité distale de cet embout de sortie, et/ou
b. l'ouverture de sortie est mise en évidence par un contraste de couleur, un composant traversé par l'ouverture de sortie présentant notamment une coloration différente de celle d'un corps de soupape visible à travers l'ouverture de sortie.
